# EUROPEAN PATENT APPLICATION

(11) **EP 0 973 034 A1**
(43) Date of publication of application: **19.01.2000**
(21) Application number: 98117861.9
(22) Date of filing: 21.09.1998
(51) Int. Cl.: G01N 33/558

(54) **Immunoassays and devices therefor**

(30) Priority: 16.07.1998 EP 98113233
(71) Applicant: Microbe Scope AG, 8803 Rüschlikon (CH)
(72) Inventor: Price, Paul C., 8810 Horgen (CH)
(74) Representative: Troesch Scheidegger Werner AG

(57) **Abstract**

The present invention refers to a one-step indirect chromatoimmunoassay to detect in a liquid sample the presence or absence of antibodies specific for a test antigen. The assay is comprising:
a) a visible tracer which is explicitly not directly specific for the antibodies being detected,
b) anti-Human Immunoglobulin G, M, A or E as the ligand component of the visible tracer when the Immunoglobulin class antibodies are to be detected, and
c) colored Latex beads as the visible substance component of the visible tracer.

## Description

The present invention relates to an assay for antibodies, and more particularly to a one-step indirect chromatoimmunoassay, to an assay arrangement and to a one-step method for determining the presence or absence of antibodies specific for a test antigen.

### Background of the invention

With the recognition that antigens bind to solid phases (e.g. nitrocellulose, polyvinyl chloride and polystyrene), the technology of immunoassays was born. Being able to capture antibodies from a liquid sample with solid-phase antigens led to the development of tracers to enable the detection of the antibodies once bound. Tracers used for immunoassays consist of a iigand component bound to a signal component. Ligands like anti-Human IgG, when labeled with signal components such as radioactive isotopes, fluorochromes or enzymes, are used in a separate reaction step to detect whether or not antibodies have bound to the immobilized antigen. Detection of the signal component initially required the use of instrumentation (radioactive isotopes or fluorochromes) or a subsequent reaction step (enzymes). The signal components were traditionally of the same order of size as the ligand component or even smaller. One or more such components were necessary to "label" the ligand. More recently, such signal components have been replaced with relatively large particles leading to the introduction of tracers visible to the unaided eye. The signal component of such visible tracers consists of a substance which is free, after sample application, to traverse along the test strip and is finally visible as one or more lines at the end of the assay process (liposomes, gold sols, dye sols and colored polymer beads like Latex are just some examples of suitable substances). The extremely large size of such components, in comparison to their predecessors, enables them to bear sufficient ligand to bind large numbers of antibodies. Calculations of the author of this invention show that under optimal conditions a single 0.3 µm latex bead which has been saturated with anti-human IgG is theoretically capable of binding 5.7 X 10⁹ molecules of IgG from a liquid sample. Even after optimal spacing of anti-human IgG on the surface of the bead in order to avoid steric hinderance, and assuming that the anti-IgG ligand components bind only one IgG molecule instead of two, one 0.3 µm latex bead is capable of binding over ten million (1.0 X 10⁷) IgG antibody molecules!

The ligand component of immunoassay tracers is normally of biological origin and is utilized to recognize an analyte in a direct or indirect manner. The first tracers to emerge used ligands such as anti-Human IgG which do not react specifically with test-antigen antibodies, but rather with all IgG antibodies in the liquid sample. For this reason, antibodies in the sample which did not bind to the antigen had to be removed with a wash procedure prior to addition of such tracers. If this were not done, the tracer would be immunochemically neutralized through binding to the tremendous excess of IgG in the sample which is not specific for test antigen. Immunoassays using such tracers are referred to as "indirect sandwich" assays, and have found wide application in the forms of radio-immunoassays, enzyme immunoassays and immunofluorescent antibody assays to name a few. Elimination of the above mentioned wash step in such an assay would require the addition of an amount of tracer adequate to bind most of the IgG antibodies present in the sample, regardless of their specificities. This would be costly, lead to elevated signal noise, and has not been seen in practice.

The author of this invention [Journal of Medical Virology 3:31-33 (1978)] was one of the first to recognize an improvement in assay performance and ease of handling when the ligand component of the tracer was (like the immobilized reagent) specific for the test analyte. In analyte-specific tracers for the detection of antibodies, the ligand is usually the antigen, an antigenic fragment or an analog of the antigen. Such assays are referred to as "direct sandwich" assays. A big advantage of using analyte-specific tracers is the possibility to eliminate the above-mentioned wash step. The use of analyte-specific visible tracers led to the introduction of one-step chromatoimmunoassays. The present state of the art would dictate that a one-step indirect chromatoimmunoassay using a tracer which is not directly specific for the analyte would either be impracticably expensive, or just not work.

As a consequence, the discovery of this inventions is the fact that the tremendous relative size of visible tracers enables even a small amount to bind a sufficient portion of IgG in the sample (approximately ten million antibodies per tracer particle), regardless of specificity, that the presence or absence of antibody specific to the test antigen can be reliably determined. This, in turn, means that even ligands which are not directly specific for the test antibody (e.g. anti-Human IgG) can be successfully employed in one-step immunoassays when bound to visible particles. To the knowledge of the author, this is novel.

### Description of the invention

This invention describes a one-step indirect chromatoimmunoassay according to the wording of claim 1 to detect in a liquid sample the presence or absence of antibodies specific for a test antigen.

Further embodiments of the invention are defined in the dependent claims. In particular, the one-step indirect chromatoimmunoassay arrangement is defined according to the wording of the claims and as described in more details in the following description.

Furthermore, a method is defined in claim 14 to detect in a liquid sample the presence or absence of antibodies specific for a test antigen.

The basic idea of the invention will be further described with reference to the attached drawings. They show:
- Fig. 1:: the upper device housing of a one-step chromatoimmunoassay arrangement according to the invention,
- Fig. 2a:: the side view of a test strip of the one-step chromatoimmunoassay arrangement according to the invention,
- Fig. 2b:: the top view of a test strip of the one-step chromatoimmunoassay arrangement according to the invention, and
- Fig. 3:: in perspective view, the lower housing of the one-step chromatoimmunoassay arrangement according to the invention.

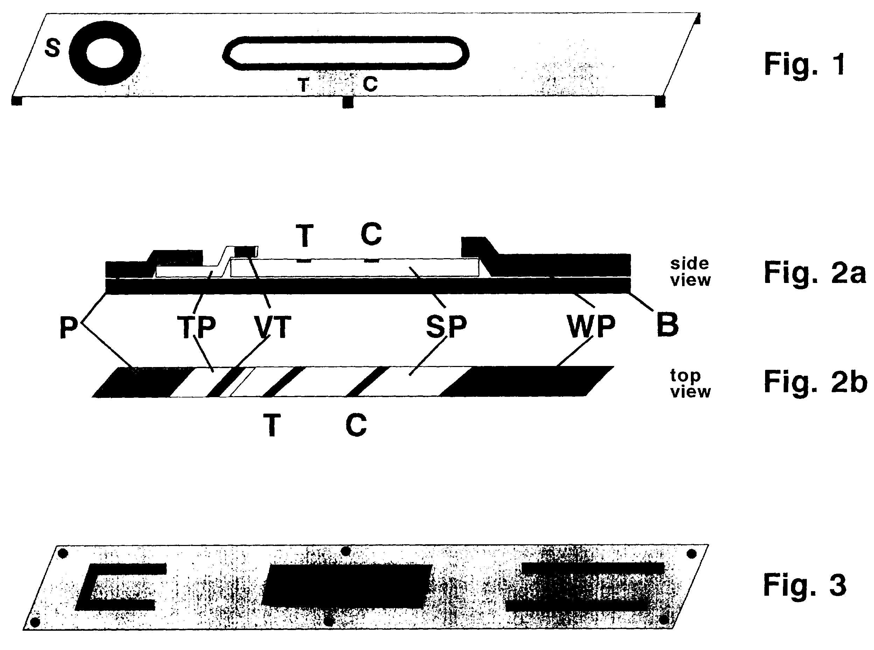

Samples are applied through aperture S of the upper housing device in fig. 1 to the sample pad P, see figs. 2a and 2b, followed by an appropriate volume of buffer solution. The diluted sample travels through the sample pad and tracer pad TP of figs. 2a and 2b, where it releases the visible tracer VT. The visible tracer is explicitly not directly specific for the antibodies being detected. Antibodies in the sample (whether specific for the test antigen or not) are bound by the visible tracer forming colored complexes as the mixture travels along the solid phase SP (in this case, nitrocellulose) of the test strip according to figs. 2a and 2b. At position T on the test strip, test antigen has been immobilized. If present in the sample, antibodies specific for the test antigen will bind at position T resulting in the formation of a colored line. If no antibodies for the test antigen are present in the sample, no colored line will appear at T. At position C on the test strip according to figs. 2a and 2b, an optional control reagent may be immobilized which binds sufficient visible tracer to result in the formation of a colored line. Finally, residual sample, visible tracer and buffer solution are adsorbed into the waste pad WP. The sample pad, tracer pad and waste pad, as well as the solid phase, are secured via adhesives in their appropriate alignments on a backing material B according to figs. 2a and 2b. The device upper and lower housings, shown in fig. 1 and fig. 3, assure proper alignment of the test strip of figs. 2a and 2b during the assay procedure and facilitate handling (lateral-flow assay). With minor or no modification, the test strip can also be used without a housing, as shown in figs. 1 and 3, in the vertical position (dip-stick assay).

Examples of similar assemblies are given in Becton Dickinson and Company EP 0 284 232 B1 and Unilever N.V. EP 0 291 194 B1.

The aspect which sets this invention apart from others is the implicit use, in a one-step immunoassay, of a visible tracer which is not directly specific for the antibodies being detected, but rather binds to antibodies in the liquid sample whether they are specific for the test antigen or not.

The present invention is advantageous in that the same visible tracer used for the detection of antibodies against one particular antigen, once optimized, can be used for detection of antibodies against many other antigens with little or no additional optimization. Being able to use the same visible tracer in assays for antibodies against differing antigens simplifies the production of such assays considerably. For example, using the present invention, assays for the detection of antibodies against various disease agents such as Rubella virus, Cytomegalovirus, Herpes Simplex virus, Varicella-Zoster virus, Measles virus, Parotitis virus, Toxoplasma gondii, Borrelia burgdorferi, Helicobacter pylori, Chlamydia, Candida and assorted other microorganismes, can be produced using the same visible tracer. Current technology requires the production and optimization of a separate "analyte-specific" visible tracer for each different disease agent assay being produced.

### Description of preferred embodiments

In a preferred embodiment of the present invention, anti-Human IgG is bound to colored Latex beads of e.g. approximately 0.3 µm diameter to form a visible tracer. This visible tracer is supported in a movable manner on e.g. a glass fiber tracer pad TP incorporated into the assay device as shown in figs. 1 to 3. Upon application of a liquid sample, the visible tracer is released from its temporary support. IgG antibodies in the sample are bound by the visible tracer forming colored complexes as the mixture travels along the solid phase (nitrocellulose) of the test strip. At position T on the test strip, test antigen has been immobilized. If present in the sample, IgG antibodies specific for the antigen will bind at position T resulting in the formation of a colored line due to the visible tracer bound to the antibodies. If no IgG antibodies specific for the antigen are present in the sample, no colored line will appear at position T.

In another preferred embodiment of the present invention, anti-Human IgM is bound to colored Latex beads to form the visible tracer. Upon application of a liquid sample, the tracer is released from its temporary support. IgM antibodies in the sample are bound forming colored complexes as the mixture travels along the test strip. If present in the sample, IgM antibodies specific for the antigen will bind at position T resulting in the formation of a colored line. If no IgM antibodies specific for the antigen are present in the sample, no colored line will appear at T.

In yet another preferred embodiment of the present invention, anti-Human IgA is bound to colored Latex beads to form the visible tracer. Upon application of a liquid sample, the tracer is released from its temporary support. IgA antibodies in the sample are bound forming colored complexes as the mixture travels along the test strip. If present in the sample, IgA antibodies specific for the antigen will bind at position T resulting in the formation of a colored line. If no IgA antibodies specific for the antigen are present in the sample, no colored line will appear at T.

In a final preferred embodiment of the present invention, anti-Human IgE is bound to colored Latex beads to form the visible tracer. Upon application of a liquid sample, the tracer is released from its temporary support. IgE antibodies in the sample are bound forming colored complexes as the mixture travels along the test strip. At position T on the test strip, test allergen has been immobilized. If present in the sample, IgE antibodies specific for the allergen will bind at position T resulting in the formation of a colored line. If no IgE antibodies specific for the allergen are present in the sample, no colored line will appear at T.

The invention will be further described with reference to the following examples:

### Example One

To produce lateral-flow assay devices for the detection of IgG antibodies against Borrelia burgdorferi, 57.5 x 185 mm mdi CNPF-PD31-L2-P25 10 µ nitrocellulose membrane laminates (Advanced Microdevices pvt Ltd., Ambala Cantt 133001 India) were sprayed using a Linomat IV (Camag, Muttenz, Switzerland) at a position 29 mm above the bottom edge with 1.0 µl/cm of a 1:20 dilution in 50 mM tris pH 8.0 buffer of Rabbit anti-Goat Immunoglobulins Z 0228 (DAKO Diagnostics AG, Zug, Switzerland) and at a position 24 mm above the bottom edge with 1.0 µl/cm of Borrelia burgdorferi antigen 7281GB (Institut Virion, Rüschlikon, Switzerland) and dried under vacuum. Using the Linomat IV, 2.0 µl/cm of anti-Human IgG covalently bound to 3.0 µm blue colored Latex beads (Sinovus Biotech Ab, Lund, Sweden) was sprayed near the front edge of 1.2 x 19 cm glass-fiber conjugate pads (Sinovus Biotech Ab, Lund, Sweden). The resulting tracer pads were also dried under vacuum. The dried tracer pads are attached (see Fig. 2) via the foreseen adhesive area to the bottom part of the membrane laminates overlapping the nitrocellulose with the conjugate facing towards the nitrocellulose. They are then covered with a 1.5 x 19 cm strip of sample pad (Sinovus Biotech Ab, Lund, Sweden) via attachment to the remaining adhesive area on the bottom part of the laminate. An absorbent pad AP120 (Advanced Microdevices pvt Ltd., Ambala Cantt 133001 India) is finally attached to the foreseen adhesive area on the top part of the membrane laminate such that it overlaps the nitrocellulose area. The above assembly is then cut into 5.0 mm wide strips which are assembled into the lower and upper housings of a device cassette (Advanced Microdevices pvt Ltd. Ambala Cantt 133001 India).

To produce dip-stick assay devices, a strip of cover tape (Advanced Microdevices pvt Ltd. Ambala Cantt 133001 India) is applied over the sample and tracer pads prior to cutting the assembled membrane laminate into 5.0 mm strips, the device cassette is not needed.

### Example Two

To produce assay devices for the detection of IgG antibodies against Helicobacter pylori, Borrelia burgdorferi antigen 7281GB (Institut Virion, Rüschlikon, Switzerland) in example one must be replaced with Helicobacter pylori antigen 7210GB (Institut Vinion, Rüschlikon, Switzerland).

### Example Three

To produce lateral-flow assay devices for the detection of IgM antibodies against Borrelia burgdorferi, the anti-Human IgG covalently bound to 0.3 µm blue colored Latex beads (Sinovus Biotech Ab, Lund, Sweden) in example one must be replaced with anti-Human IgM covalently bound to 0.3 µm red colored Latex beads (Sinovus Biotech Ab, Lund, Sweden).

In the lateral-flow examples above, 20 µl of human serum or plasma, or 40 µl of human fresh full blood containing anticoagulant are applied directly to the sample pad, followed by 100 µl of sample buffer (Sinovus Biotech Ab, Lund, Sweden). In the dip-stick examples, similar sample and buffer volumes are pre-mixed in small vessels into which the test strip is latter dipped. The appearance of a line at position "C" must be seen for all tests to be valid. Appearance of a line at "T" is indicative of the presence of the test antibody.

As is seen from the above examples, simply replacing the antigen allows the use of a single tracer pad in one-step assays for the detection of antibodies against various antigens. This is valid for the different classes of antibodies (IgG, IgA, IgM and IgE).

The present invention is of course not restricted to the given preferred embodiments and to the mentioned Examples, as they only serve to describe the invention in more detail and enable a better understanding of it. The basic idea of the invention is that the chromatoimmunoassay arrangement is designed such that in a liquid sample presence or absence of antibodies specific for a test antigen may be detected utilizing:
a) a visible tracer, which is explicitly not directly specific for the antibodies being detected,
b) anti-Human IgG as the ligand component of the visible tracer when IgG class antibodies are to be detected, and
c) colored Latex beads as the visible substance component of the visible tracer.

## Claims

1. A one-step indirect chromatoimmunoassay to detect, in a liquid sample, the presence or absence of antibodies specific for the test antigen comprising:
a) a visible tracer which is explicitly not directly specific for the antibodies being detected,
b) an anti-Human Immunoglobulin as the ligand component of the visible tracer when the Immunoglobulin class antibodies are to be detected,
c) colored Latex beads as the visible substance component of the visible tracer.

2. An assay as claimed in particular in claim 1, wherein anti-Human Immunoglobulin G is the ligand component of the visible tracer when Immunoglobulin G class antibodies are to be detected.

3. An assay as claimed in particular in one of the claims 1 or 2, wherein anti-Human Immunoglobulin M is the ligand component of the visible tracer when Immunoglobulin M class antibodies are to be detected.

4. An assay as claimed in particular in one of the claims 1 or 2, wherein anti-Human Immunoglobulin A is the ligand component of the visible tracer when Immunoglobulin A class antibodies are to be detected.

5. An assay as claimed in particular in one of the claims 1 or 2, wherein anti-Human Immunoglobulin E is the ligand component of the visible tracer when Immunoglobulin E class antibodies are to be detected.

6. An assay as claimed in particular in one of the claims 1 to 5, wherein a mixture of anti-Human Immunoglobulin G, A and M anti-Human Immunoglobulin or a generic antibody binder such as Protein A is the ligand component of the visible tracer when total antibodies are to be detected.

7. An assay as claimed in particular in one of the claims 1 to 6, wherein the Latex beads are replaced with gold particles.

8. An assay as claimed in particular in one of the claims 1 to 6, wherein the Latex beads are replaced with another detectable particulate substance.

9. An assay arrangement comprising a test strip with at least three portions, such as
a) a sample pad
b) a tracer pad, and
c) a solid phase,
of which test strip at least two portions have a zone of overlapping in which one pad is overlapping the neighbor pad.

10. An assay arrangement, in particular according to claim 9, wherein at least two zones of overlapping are present.

11. An assay arrangement, in particular according to one of the claims 9 or 10, wherein a fourth portion is present such as a waste pad, which is arranged next to the solid phase.

12. An assay arrangement, in particular according to one of the claims 9 to 11, wherein the test strip is obtainable by arranging a sample pad next to the tracer pad, overlapping the tracer pad, the tracer pad is arranged next to the solid phase, at least partially overlapping the solid phase and wherein optionally next to the solid phase a waste pad is arranged, the waste pad is overlapping at least partially the solid phase.

13. An assay arrangement, in particular according to one of the claims 9 to 12, wherein the test strip is arranged within a housing, consisting of an upper housing and a lower housing, in which upper housing at least two openings are arranged, one opening is arranged above the sample pad and the other opening is longitudinally extended and is arranged over the solid phase.

14. Method to detect in a liquid sample the presence or absence of antibodies specific for a test antigen with a one-step indirect chromatoimmunoassay, comprising the steps of applying a sample on a sample pad, followed by adding an appropriate volume of buffer solution, the diluted sample travels through a sample pad and tracer pad, where it releases the visible tracer, the visible tracer is explicitly not directly specific for the antibodies being detected, the antibodies in the sample are bound by the visible tracer forming colored complexes as the mixture travels along the solid phase of the test strip, on the test strip the antigen has been immobilized and if present in the sample, antibodies specific for the test antigen will bind on the solid phase resulting in the formation of a colored line.
